(19) Europäisches Patentamt
European Patent Office
Office européen des brevets

(11) Veröffentlichungsnummer: **0 103 274**
**A2**

# (12) EUROPÄISCHE PATENTANMELDUNG

(21) Anmeldenummer: **83108856.2**

(22) Anmeldetag: **08.09.83**

(51) Int. Cl.[3]: **C 07 C 103/153**, C 07 C 103/58, C 07 C 103/60, C 07 C 103/64, C 07 C 103/66, C 07 D 307/52, C 07 D 295/18, C 07 C 143/675, C 07 C 121/43, C 07 C 121/78, D 06 M 13/40 // C07C69/60

(30) Priorität: **11.09.82 DE 3233830**

(43) Veröffentlichungstag der Anmeldung: **21.03.84 Patentblatt 84/12**

(84) Benannte Vertragsstaaten: **AT BE CH DE FR GB IT LI NL SE**

(71) Anmelder: **HOECHST AKTIENGESELLSCHAFT, Postfach 80 03 20, D-6230 Frankfurt am Main 80 (DE)**

(72) Erfinder: **Schinzel, Erich, Dr., Ostpreussenstrasse 43, D-6238 Hofheim am Taunus (DE)**
Erfinder: **Kleber, Rolf, Dr., Am Trieb 41, D-6078 Neu-Isenburg (DE)**

(54) **Perfluoralkyl-malein- und -fumarsäureamide, Verfahren zu deren Herstellung und ihre Verwendung als schmutzabweisendes Mittel.**

(57) Perfluoralkyl-malein- und -fumarsäureamide der Formel

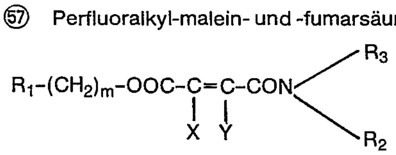

worin $R_1$ $C_2$–$C_{20}$-Perfluoralkyl oder Perfluoralkoxyperfluoralkyl, $R_2$ Wasserstoff, gegebenenfalls substituiertes Alkyl, Alkenyl, Alkinyl, Cyclohexyl, Phenyl, Naphthyl oder Phenylamino, $R_3$ Wasserstoff, gegebenenfalls substituiertes Alkyl oder Cyclohexyl, $R_2$ und $R_3$ gemeinsam mit dem Stickstoffatom einen fünf- oder sechsgliedrigen hydrierten Heterocyclus, X und Y Wasserstoff, Halogen oder $C_1$–$C_6$-Alkyl und m eine ganze Zahl von 1 bis 6 bedeuten, Verfahren zu deren Herstellung und deren Verwendung zur öl- und wasserabweisenden Ausrüstung von Textilmaterial.

EP 0 103 274 A2

HOECHST AKTIENGESELLSCHAFT HOE 82/F 182 Dr.OT/sch

Perfluoralkyl-malein- und -fumarsäureamide, Verfahren zu deren Herstellung und ihre Verwendung als schmutzabweisendes Mittel

Die Erfindung betrifft neue Perfluoralkylverbindungen, die sich zur schmutzabweisenden Ausrüstung von Fasern oder Geweben aus synthetischen, halbsynthetischen oder natürlichen Materialien, vorzugsweise aus Polyethylenterephthalat oder Polyamiden eignen.

Verbindungen, die Perfluoralkylreste enthalten sind als schmutzabstoßende Mittel bereits bekannt. So werden in der DE-OS 2 628 776 Verbindungen beschrieben, die im wesentlichen aus wenigstens einer fluorierten Verbindung mit wenigstens einem Benzolring bestehen. Ferner kennt man schmutzabstoßende Mittel, die aus polymeren Verbindungen, die fluorierte Gruppen enthalten, aufgebaut sind. Speziell sind fluorierte Verbindungen in der US-PS 3 547 861 beschrieben, die fluorierte Acrylate und Polyacrylate betrifft, worin der fluorierte Rest sich von einem fluorierten Alkohol mit einer endständigen fluorierten Alkoxygruppe herleitet. Ähnliche Produkte für solche Verwendungen sind ebenfalls bekannt, worin der fluorierte Rest des Polyacrylats ein geradkettiger fluorierter Alkohol ist.

Die erfindungsgemäßen Verbindungen entsprechen der allgemeinen Formel (1)

$$R_1 - (CH_2)_m - OOC - \underset{X}{\underset{|}{C}} = \underset{Y}{\underset{|}{C}} - CON\begin{matrix} R_3 \\ R_2 \end{matrix} \qquad (1)$$

in welcher

$R_1$ Perfluoralkyl oder Perfluoralkoxy-perfluoralkyl mit jeweils insgesamt 2 - 20, vorzugsweise 4 - 14 C-Atomen,

$R_2$ Wasserstoff, geradkettiges oder verzweigtes $C_1$-$C_{18}$-Alkyl, das durch Hydroxylgruppen, eine Amino-, Alkylamino-,

Diethylamino- oder Trialkylammoniumgruppe, eine Acylaminogruppe, eine Aminoalkylaminogruppe, eine Acylaminoalkylaminogruppe, eine Alkoxy-, Hydroxyalkoxy-, Hydroxyalkylthiogruppe, eine Acyloxygruppe, 1 - 2 Carboxyl- oder funktionell abgewandelte Carboxylgruppen, eine Sulfo- oder funktionell abgewandelte Sulfogruppe, eine Phenyl-, Phenoxy- oder Furanylgruppe substituiert sein kann; $C_3$-$C_{18}$-Alkenyl, das durch Hydroxylgruppen substituiert sein kann; $C_3$-$C_8$-Alkinyl, das durch Hydroxylgruppen substituiert sein kann; Cyclohexyl, Phenyl, das durch 1 - 3 $C_1$-$C_8$-Alkylgruppen, 1 oder 2 Halogenatome, vorzugsweise Chloratome, eine Hydroxylgruppe, eine Amino-, Alkylamino-, Dialkylamino- oder Trialkylammoniumgruppe, eine Acylaminogruppe, eine Alkoxygruppe, eine Acyloxygruppe, 1 - 2 Carboxyl- oder funktionell abgewandelte Carboxylgruppen, 1 - 2 Sulfo- oder funktionell abgewandelte Sulfogruppen substituiert sein kann; α- oder ß-Naphthyl, das durch 1 - 3 Sulfogruppen substituiert sein kann; Phenylamino, das durch eine Sulfogruppe substituiert sein kann;

$R_3$ Wasserstoff, geradkettiges oder verzweigtes $C_1$-$C_8$-Alkyl, das durch Hydroxylgruppen, eine Sulfogruppe oder einen Phenylrest substituiert sein kann; Cyclohexyl,

$R_2$ und $R_3$ gemeinsam mit dem Stickstoffatom den Pyrrolidinring, Morpholinring, Piperidinring, Piperazinring oder Piperaziniumring,

X und Y Wasserstoff, Halogen oder $C_1$-$C_6$-Alkyl und

m eine ganze Zahl von 1 bis 6, vorzugsweise 1 bis 4

bedeuten.

Bevorzugt sind die Perfluoralkylverbindungen der Formel (1), in welcher

$R_1$ eine Gruppe der Formel $C_lF_{2l+1}$-, eine Gruppe der Formel $H(C_2F_4)_n$- oder eine Gruppe der Formel $(CH_3)_2CFO(CF_2)_o$-

l die Zahlen 6, 8, 10, 12, 14,
n die Zahlen 1, 2, 3 und 4 und
o ganze Zahlen von 2 bis 8,
$R_2$ Wasserstoff, geradkettiges oder verzweigtes $C_1$-$C_8$-Alkyl, das durch Hydroxylgruppen, eine Amino-, Alkylamino-, Dialkylamino- oder Trialkylammoniumgruppe, eine Hydroxylalkoxygruppe, eine Carboxy- oder eine Sulfogruppe substituiert sein kann; Phenyl, das durch eine Hydroxylgruppe, eine Alkoxygruppe, eine Dialkylamino- oder Trialkylammoniumgruppe, eine Carboxy- oder 1-2 Sulfo-Gruppen substituiert sein kann; α- oder ß-Naphthyl-, das durch 1-3 Sulfogruppen substituiert sein kann, eine Phenylaminogruppe,
$R_3$ Wasserstoff, geradkettiges oder verzweigtes $C_1$-$C_8$-Alkyl, das durch Hydroxylgruppen substituiert sein kann,
$R_2$ und $R_3$ gemeinsam mit dem Stickstoffatom den Morpholin-, Piperidin-, Piperazin- oder Piperaziniumring und
X und Y Wasserstoff, die vorzugsweise in Transstellung angeordnet sind,
bedeuten.

Der Begriff "Perfluoralkyl" bzw. "Perfluoralkoxy" umfaßt sowohl solche Gruppen mit endständigen $-CF_3$ als auch mit endständigen $-CF_2H$ Gruppen. Die Begriffe "funktionell abgewandelte Carboxygruppen" und "funktionell abgewandelte Sulfogruppen" umfassen Cyano-, Carbonsäureester-, Carbonamid-, mono- und di-Alkylcarbonamidgruppen bzw. Sulfonsäureester-, Sulfonamid-, mono- und di-Alkylsulfonamidgruppen. Alkyl- und Alkoxygruppen enthalten, soweit nicht anders definiert, 1 bis 4 C-Atome. Unter einer Acylgruppe ist im wesentlichen eine $C_1$-$C_4$ Alkanoylgruppe zu verstehen. Die Carboxy- und Sulfogruppen können auch in Form der Salze mit farblosen Kationen vorliegen, beispielsweise als Ammonium-, Kalium- oder Natriumsalze.

Die erfindungsgemäßen Perfluoralkylamide sind schmutzabweisende Verbindungen, die gegenüber Wasser und Öl ein

hohes Abweisungsvermögen besitzen und die auch nach wiederholten Wäschen und Trockenreinigungen auf der Faser verbleiben. Ein weiterer Vorteil der erfindungsgemäßen Verbindungen besteht darin, daß sie in Lösung oder als Dispersion auf die Fasermaterialien aufgebracht werden können oder sich durch Vermischen mit Pellets des thermoplastischen Kunststoffs und anschließendes Verformen zu Fasern oder Fäden einarbeiten lassen. Ein spezieller Vorteil der neuen schmutzabstoßenden Mittel besteht auch darin, daß sie ein einwandfreies Anfärben der Fasern oder Fäden, worin diese Mittel eingearbeitet werden, gestatten. Die neuen schmutzabstoßenden Mittel können auch mit zufriedenstellenden Ergebnissen zusammen mit einem Farbstoff aus einem Bade aufgebracht werden.

Die Herstellung der erfindungsgemäßen Verbindungen (1) erfolgt durch Umsetzung eines Maleinsäureanhydrids mit 1 Mol eines perfluorierten Alkohols oder Alkoxyalkohols, gegebenenfalls in Gegenwart eines inerten organischen Lösungsmittels, wie Dimethylformamid, N-Methylpyrrolidon, Hexamethylphosphorsäuretriamid, Tetramethylharnstoff, Toluol, Chlorbenzol oder Dichlorbenzol bei Temperaturen von 30 bis 130°C, vorzugsweise bei 40 bis 70°C. Diese Reaktion kann durch Zugabe eines tert. Amins, wie Triethylamin, N,N-Dimethylanilin, Pyridin, N-Methyl-pyridin katalytisch beschleunigt werden.

Die gebildeten Maleinsäure- und/oder Fumarsäure-perfluoralkyl-halbester können in üblicher Weise durch Reaktion mit Phosphorchloriden, Phosgen oder Thionylchlorid in die entsprechenden Malein- und/oder Fumarsäureperfluoralkyl-halbester-chloride verwandelt werden, die sich mit geeigneten Aminen, zweckmäßig in Gegenwart von Protonenfängern wie tert. Aminen, zu den Verbindungen der Formel (1) umsetzen lassen. Die letztgenannte Reaktion erfolgt durch Erhitzen auf ca. 50 - 60°C in einem inerten polaren organischen Lösungsmittel.

Die mit Diaminen, wie Diethylamino-ethylamin, 3-Dimethylaminoanilin oder N-ß-Hydroxyethyl-piperazin erhaltenen basischen Amide können in inerten organischen Lösungsmitteln mit Alkylierungsmitteln, wie Dimethylsulfat, p-Toluolsulfonsäuremethylester oder Alkylhalogeniden zu Ammoniumamiden weiter umgesetzt werden.

Die erfindungsgemäßen Verbindungen der Formel (1) eignen sich zur gleichzeitigen Hydrophobierung und Oleophobierung von synthetischen und natürlichen Fasern und Geweben, insbesondere für Polyester, Polyamid, Polyacrylnitril und Wolle. Die Applikation dieser Verbindungen auf das Textilmaterial erfolgt nach bekannten Verfahren durch Imprägnieren mit einer Lösung der Verbindungen der Formel (1) in Wasser oder einem geeigneten organischen Lösungsmittel, vorzugsweise in Aceton oder Dimethylformamid. Man kann die Verbindungen der Formel (1) aber auch in Form wäßriger Dispersionen einsetzen. Das Textilmaterial wird nach dem Imprägnieren abgequetscht, getrocknet und thermofixiert. Besonders bevorzugt ist die gleichzeitige Mitverwendung der Verbindungen der Formel (1) in einem üblichen Faserpräparationsmittel. Die Auflage der Verbindungen der Formel (1) auf dem Textilmaterial beträgt im allgemeinen 0,05 bis 1, vorzugsweise 0,1 bis 0,4 Gew.-%, berechnet auf Gehalt an Fluor in den Verbindungen der Formel (1).

Es wird angenommen, daß die schmutzabstoßenden Eigenschaften den Thermoplasten durch die vorliegenden Verbindungen der Formel (1) auf Grund ihrer Funktion verliehen werden, die Oberflächenenergie der Thermoplasten zu vermindern. Dieser Effekt kann durch ein Tempern bei Temperaturen oberhalb der Glasübergangstemperatur der Thermoplasten und unterhalb der Zersetzungstemperatur sowohl der Thermoplasten als auch des schmutzabstoßenden Mittels verbessert werden. Geeignete Zeiten für ein solches Tempern liegen im Bereich von etwa 1 bis 240 Minuten. Die Temperaturen des Temperns liegen typischerweise bei etwa 100 bis 220°C.

Eine weitere Verbesserung des Effektes der erfindungsgemäßen schmutzabstoßenden Mittel, die eine Hydroxylgruppe in dem Amidrest enthalten, bekommt man bei Mitverwendung eines difunktionellen oder trifunktionellen Epoxids oder Isocyanats in dem flüssigen Medium, das das schmutzabstoßende Mittel enthält und in welches die Faser oder der andere thermoplastische Gegenstand eingetaucht wird oder mit der diese besprüht oder anderweitig behandelt werden, und zwar zusammen mit einem Katalysator, wie einem Amin, um die Reaktion der Hydroxylgruppe mit einer Epoxydgruppe oder Isocyanatgruppe beim anschließenden Tempern zu fördern.

Herstellungsbeispiele

Tabelle 1

$C_lF_{2l+1}CH_2CH_2OOC-CH=CH-CO-$ =A-
trans
l = 6, 8, 10, 12, (14)

| Lfd. Nr. | Formel | Methode | Ausb. bez. auf Säurechlorid | Analyse % F |
|---|---|---|---|---|
| (101) | $A-NH_2$ | A | 96,1 % | Ber. 57,1<br>Gef. 56,1 |
| (102) | $A-NHCH_3$ | A | 87,8 % | Ber. 55,7<br>Gef. 54,5 |
| (103) | $A-NHCH(CH_3)_2$ | B | 94,1 % | Ber. 53,1<br>Gef. 52,6 |
| (104) | $A-NH(CH_2)_5CH_3$ | C | 81,9 % | Ber. 49,5<br>Gef. 50,5 |
| (105) | $A-NH(CH_2)_{11}CH_3$ | C | 79,5 % | Ber. 43,7<br>Gef. 42,9 |
| (106) | $A-NH(CH_2)_{17}CH_3$ | C | 94,5 % | Ber. 39,1<br>Gef. 37,9 |
| (107) | $A-NHCH_2CH=CH_2$ | B | 84,0 % | Ber. 53,2<br>Gef. 53,2 |
| (108) | $A-NH(CH_2)_8CH=CH(CH_2)_7CH_3$ | C | 79,3 % | Ber. 39,2<br>Gef. 38,4 |
| (109) | $A-N(CH_3)_2$ | D | 75,0 % | Ber. 54,3<br>Gef. 54,1 |
| (110) | $A-N[CH(CH_3)_2]_2$ | B | 78,7 % | Ber. 49,5<br>Gef. 50,7 |
| (111) | $A-NHCH_2CH_2NH_2$ | E | 78,2 % | Ber. 52,9<br>Gef. 51,8 |

Tabelle 1 (Fortsetzung)

| Lfd. Nr. | Formel | Methode | Ausb. bez. auf Säurechlorid | Analyse % F |
|---|---|---|---|---|
| (112) | A-NH$(CH_2)_6$NH-A | F | 81,9 % | Ber. 53,2<br>Gef. 53,2 |
| (113) | A-$NHCH_2CH_2NHCH_2CH_2$NH-A | F | 54,9 % | Ber. 53,7<br>Gef. 52,4 |
| (114) | A-$NHCH_2CH_2OH$ | G | 68,6 % | Ber. 52,9<br>Gef. 51,6 |
| (115) | A-$NHCH_2C(CH_3)_2CH_2OH$ | G | 88,1 % | Ber. 49,4<br>Gef. 49,4 |
| (116) | A-$NHCH_2CH_2OCH_2CH_2OH$ | G | 79,3 % | Ber. 49,2<br>Gef. 50,4 |
| (117) | A-$NCH_2CH_2OH$<br>$CH_2$ | G | 86,4 % | Ber. 51,7<br>Gef. 51,2 |
| (118) | A-$N(CH_2CH_2OH)_2$ | G | 57,2 % | Ber. 49,2<br>Gef. 50,4 |
| (119) | A-$N(CH_2CHOHCH_3)_2$ | G | 74,5 % | Ber. 47,2<br>Gef. 48,2 |
| (120) | A-$NHCH_2CH_2CH_2OCH_3$ | G | 75,1 % | Ber. 50,5<br>Gef. 51,1 |
| (121) | A-$NHCH_2COOH$ | H | 88,8 % | Ber. 51,7<br>Gef. 51,9 |
| (122) | A-$NHC(CH_3)_2COOH$ | H | 77,8 % | Ber. 49,4<br>Gef. 50,7 |
| (123) | A-N-$CH_2CH_2CN$<br>$CH_3$ | G | 84,2 % | Ber. 50,9<br>Gef. 51,1 |

Tabelle 1 (Fortsetzung)

| Lfd. Nr. | Formel | Methode | Ausb. bez. auf Säurechlorid | Analyse % F |
|---|---|---|---|---|
| (124) | A-NCH$_2$COOH (N-CH$_3$) | H | 83,4 % | Ber. 50,5<br>Gef. 51,4 |
| (125) | 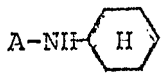 | G | 91,6 % | Ber. 49,7<br>Gef. 49,6 |
| (126) | 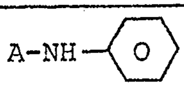 | G | 93,2 % | Ber. 50,2<br>Gef. 49,9 |
| (127) | 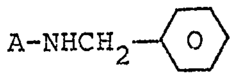 | G | 89,6 % | Ber. 49,1<br>Gef. 49,0 |
| (128) | 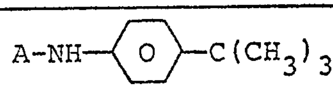 | G | 96,9 % | Ber. 46,1<br>Gef. 45,6 |
| (129) | 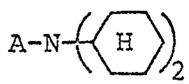 | G | 91,0 % | Ber. 44,0<br>Gef. 44,0 |
| (130) | 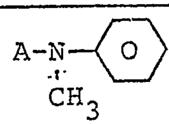 | G | 93,1 % | Ber. 49,1<br>Gef. 48,5 |
| (131) | 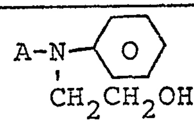 | G | 89,6 % | Ber. 46,9<br>Gef. 48,0 |
| (132) | 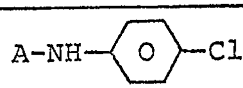 | G | 91,6 % | Ber. 47,6<br>Gef. 47,3 |

Tabelle 1 (Fortsetzung)

| Lfd. Nr. | Formel | Methode | Ausb. bez. auf Säurechlorid | Analyse % F |
|---|---|---|---|---|
| (133) | 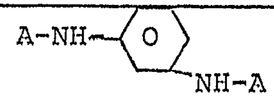 | F | 95,0 % | Ber. 53,5<br>Gef. 52,6 |
| (134) | 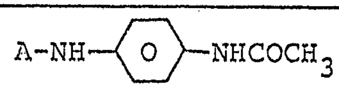 | G | 91,5 % | Ber. 46,0<br>Gef. 46,2 |
| (135) | 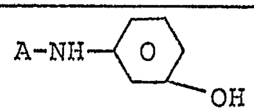 | G | 90,4 % | Ber. 48,9<br>Gef. 48,9 |
| (136) | 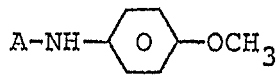 | G | 92,4 % | Ber. 47,9<br>Gef. 47,0 |
| (137) | 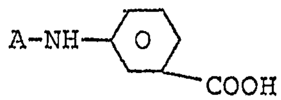 | H | 87,7 % | Ber. 46,9<br>Gef. 47,4 |
| (138) | 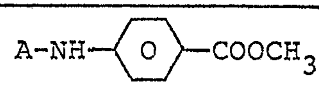 | G | 92,8 % | Ber. 45,9<br>Gef. 45,6 |
| (139) | 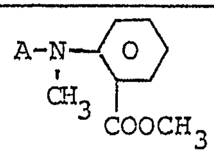 | G | 86,9 % | Ber. 45,0<br>Gef. 45,0 |
| (140) | 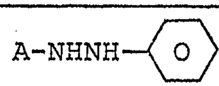 | G | 91,2 % | Ber. 49,0<br>Gef. 48,8 |
| (141) | 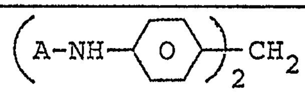 | F | 93,0 % | Ber. 49,7<br>Gef. 48,7 |

Tabelle 1 (Fortsetzung)

| Lfd. Nr. | Formel | Methode | Ausb. bez. auf Säurechlorid | Analyse % F |
|---|---|---|---|---|
| (142) | 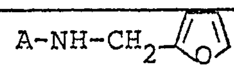 | G | 86,9 % | Ber. 49,9<br>Gef. 50,4 |
| (143) | 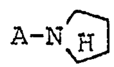 | G | 85,6 % | Ber. 52,0<br>Gef. 51,1 |
| (144) | 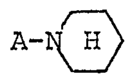 | G | 66,8 % | Ber. 50,8<br>Gef. 50,8 |
| (145) | 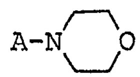 | G | 82,7 % | Ber. 50,7<br>Gef. 50,2 |
| (146) | 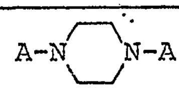 | F | 98,2 % | Ber. 54,6<br>Gef. 53,4 |

Tabelle 1 (Fortsetzung)

| Lfd. Nr. | Formel | Methode | Ausb. bez. auf Säurechlorid | Analyse % F | % N |
|---|---|---|---|---|---|
| (150) | 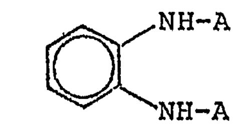 | F | 89,1 % | Ber. 53,<br>Gef. 52,1 | 2,4<br>2,8 |
| (151) | 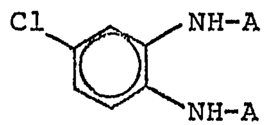 | F | 96,9 % | Ber. 51,8<br>Gef. 51,5 | 2,3<br>2,7 |
| (152) | 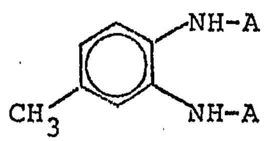 | F | 92,8 % | Ber. 52,7<br>Gef. 51,8 | |
| (153) | 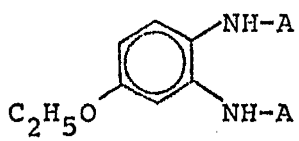 | F | 94,3 % | Ber. 51,4<br>Gef. 51,3 | |
| (154) | 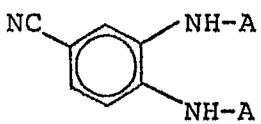 | F | 98,1 % | Ber. 52,2<br>Gef. 51,9 | |
| (155) | 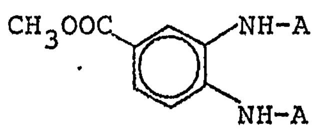 | F | 94,0 % | Ber. 50,8<br>Gef. 50,1 | 2,3<br>2,5 |
| (156) | 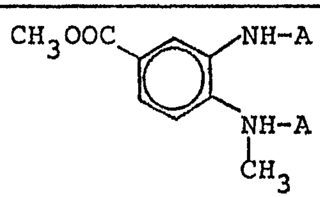 | F | 97,5 % | Ber. 50,2<br>Gef. 49,6 | 2,3<br>2,9 |

Tabelle 1 (Fortsetzung)

| Lfd. Nr. | Formel | Methode | Ausb. bez. auf Säurechlorid | Analyse % F | % N |
|---|---|---|---|---|---|
| (157) | 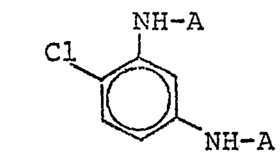 | F | 97,0 % | Ber. 51,8<br>Gef. 50,9 | 2,3<br>2,8 |
| (158) | 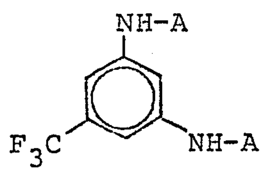 | F | 95,0 % | Ber. 55,0<br>Gef. 54,2 | |
| (159) | 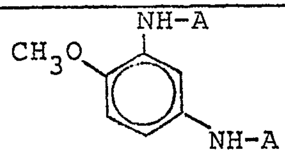 | F | 91,9 % | Ber. 52,0<br>Gef. 51,1 | |
| (160) | 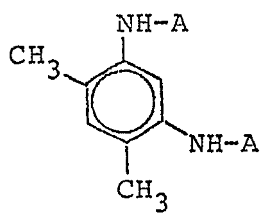 | F | 93,7 % | Ber. 52,0<br>Gef. 51,8 | |
| (161) | 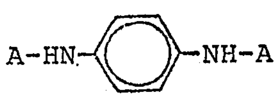 | F | 89,1 | Ber. 53,3<br>Gef. 52,4 | 2,4<br>2,9 |

Tabelle 2

$C_lF_{2l+1}CH_2CH_2OOC-CH=CH-CO-$ =A-
trans
l = 6, 8, 10, 12, (14)

| Lfd.Nr. | Formel | Methode | Ausbeute | Analyse % F | Analyse % N,S |
|---|---|---|---|---|---|
| (201) | $A-NHCH_2CH_2N(C_2H_5)_2$ | I | 81,5 % | Ber. 48,4<br>Gef. 48,1 | 4,32 (N)<br>3,6 |
| (202) | $A-NHCH_2CH_2\overset{(+)}{N}(C_2H_5)_2$ $CH_3$ $CH_3OSO_3^{(-)}$ | J | 70,8 % | | Ber. 4,14 (S)<br>Gef. 4,1 |
| (203) | $A-NHCH_2CH_2CH_2N(CH_3)_2$ | I | 79,5 % | Ber. 49,5<br>Gef. 48,5 | 4,41 (N)<br>4,1 |
| (204) | $A-NHCH_2CH_2CH_2N(CH_3)_3CH_3OSO_3^{(-)}$ | J | 69,7 % | | Ber. 4,21 (S)<br>Gef. 3,9 |
| (205) | A-NH— 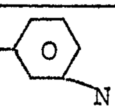 $N(CH_3)_2$ | I | 95,0 % | Ber. 46,96<br>Gef. 46,6 | 4,19 (N)<br>4,1 |
| (206) | A-NH— 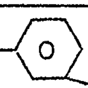 (+) $N(CH_3)_3$ $CH_3OSO_3^{(-)}$ | J | 77,6 % | | Ber. 4,03 (S)<br>Gef. 4,0 |
| (207) | A-NH— 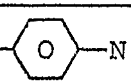 —$N(C_2H_5)_2$ | I | 89,8 % | Ber. 45,07<br>Gef. 45,0 | 4,02 (N)<br>3,8 |
| (208) | A- 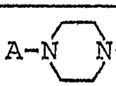 -$CH_2CH_2OH$ | I | 76,9 % | Ber. 47,38<br>Gef. 48,1 | 4,22 (N)<br>3,9 |

<u>Tabelle 3</u>

$C_1F_{2l+1}CH_2CH_2OOC-CH=CH-CO-$ =A-
trans

l = 6, 8, 10, 12, (14)

| Lfd.Nr. | Formel | Methode | Ausbeute | Analyse % F |
|---|---|---|---|---|
| (301) | $A-NHCH_2CH_2SO_3Na$ | K | 87,8 % a) | Ber. 46,2<br>Gef. 44,6 |
| (302) | $A-N(CH_3)CH_2CH_2SO_3Na$ | K | 100 % a) | Ber. 45,3<br>Gef. 43,8 |
| (303) | 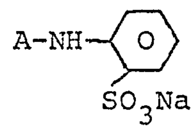 | K | 82,1 % b) | Gef. 34,0 |
| (304) | 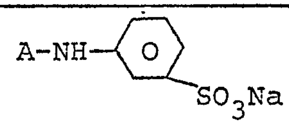 | K | 92,7 % a) | Ber. 43,15<br>Gef. 41,0 |
| (305) | 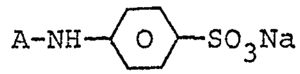 | K | 85,1 % b) | Gef. 35,2 |
| (306) | 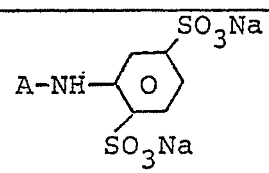 | K | 84,5 % b) | Gef. 28,1 |
| (307) | 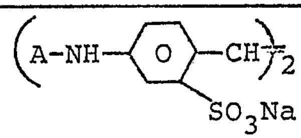 | K | 82,5 % b) | Gef. 32,7 |

Tabelle 3 (Fortsetzung)

| Lfd.Nr. | Formel | Methode | Ausbeute | Analyse % F |
|---|---|---|---|---|
| (308) | 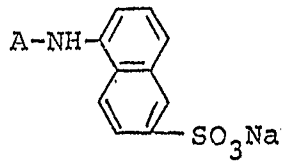 | K | 86,6 % b) | Gef. 36,6 |
| (309) | 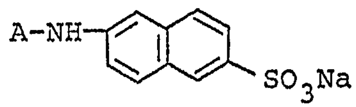 | K | 97,2 % a) | Ber. 40,4<br>Gef. 38,4 |
| (310) | 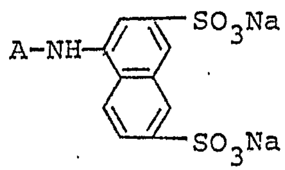 | K | 85,8 % b) | Gef. 26,8 |
| (311) | 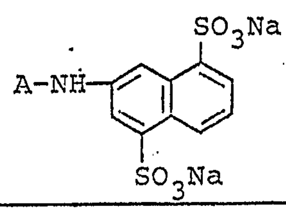 | K | 67,2 % b) | Gef. 21,0 |
| (312) | 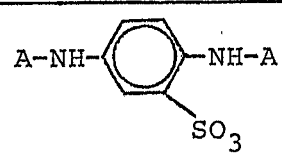 | K | 89,9 % c) | Ber. 49,9<br>Gef. 48,9 |

a) Ermittelt aus dem Natriumchlorid- und Wassergehalt.
b) Ermittelt aus dem Fluorgehalt.
c) Es wurden 0,3 Mol Säurechlorid eingesetzt und das Reaktionsprodukt durch Ansäuern mit konz. Salzsäure abgeschieden.

Methode A:

Zu einer Mischung aus 1 Mol des Perfluoralkylalkohols $C_lF_{2l+1}CH_2CH_2OH$, l=6,8,10,12 und 1 Mol Maleinsäureanhydrid in Toluol wurden 0,06 Mol Triethylamin eingetropft. Anschließend wurde auf 50-55° erhitzt und 6 Stunden bei dieser Temperatur verrührt. Der ausgefallene Halbester wurde bei Raumtemperatur abgesaugt, 3mal mit Toluol gewaschen und bei 50°C im Vakuum getrocknet. Bei der Titration in Ethanol/Wasser gegen Phenolphthalein wurde (unter Berücksichtigung des zugesetzten Triethylamins) das erwartete Äquivalentgewicht gefunden.
Ausbeute: 97,3 %

Thionylchlorid wurde in ca. 20 %igem Überschuß in Toluol vorgelegt und auf 50° angeheizt. Bei dieser Temperatur wurde in ca. 2 Stunden der obige Halbester eingetragen und die gebildete klare, farblose Lösung 10 Stunden bei 50° nachgerührt. Das Reaktionsgemisch wurde im Vakuum bei 50 - 60° bis zur Gewichtskonstanz eingedampft.
Ausbeute: 100 %

In 100 ml Aceton wurden bei 5° 12g Ammoniak bzw. Amin eingeleitet. Zu dieser Lösung wurden 0,15 Mol des obigen Säurechlorids in Form einer acetonischen Lösung in 20 Minuten zugesetzt und das Reaktionsgemisch durch weiteres Einleiten von Ammoniak bzw. Amin bei pH 10 gehalten. Die gebildete Suspension wurde 1/2 Stunde bei Raumtemperatur nachgerührt, anschließend zum Sieden erhitzt und 4 Stunden unter Rückflußsieden gerührt. Das Lösungsmittel wurde abdestilliert, der Rückstand mit 400 ml Wasser versetzt und bei 50° ausgerührt. Nach dem Abkühlen auf Raumtemperatur wurde abgesaugt, das Nutschgut frei von Chlorionen gewaschen und bei 60° im Vakuum getrocknet.

Methode B:

0,315 Mol Amin wurden in 50 ml Aceton gelöst und bei Raumtemperatur mit 0,15 Mol des Säurechlorids aus Methode A in

Form einer acetonischen Lösung tropfenweise versetzt. Die gebildete Suspension wurde 3 - 6 Stunden am Rückfluß gekocht, das Lösungsmittel abdestilliert und der Rückstand mehrmals mit je 400 ml Wasser (bei 50°) verrührt. Das halbfeste Reaktionsprodukt wurde abgesaugt und mit Wasser neutral und frei von Chlorionen gewaschen.
Trocknung bei 60° im Vakuum.

Methode C:

0,15 Mol Amin wurden in ca. 100 ml Aceton gelöst und mit 0,165 Mol Pyridin versetzt. Bei Raumtemperatur wurden 0,15 Mol des Säurechlorids aus Methode A in Form einer acetonischen Lösung zugetropft und das Reaktionsgemisch 3 - 6 Stunden am Rückfluß gekocht. Nach dem Abdestillieren des Lösungsmittels wurde der Rückstand mehrmals mit je 400 ml Wasser (bei 50°) ausgerührt, bis im abdekantierten Waschwasser keine Chlorionen mehr nachweisbar waren. Es wurde bei 60° im Vakuum getrocknet.

Methode D:

Es wurde wie bei Methode A angegeben gearbeitet. Da sich das Reaktionsprodukt nur schlecht von der wäßrigen Phase abtrennen ließ, wurden 100 ml Essigsäureethylester und 40 g Natriumchlorid zugegeben. Nach dem Verrühren wurde die sich gut absetzende organische Phase abgetrennt, mehrmals mit Wasser bis zur völligen Entfernung von Chlorionen gewaschen, mit Natriumsulfat getrocknet und im Vakuum bis zur Gewichtskonstanz eingedampft.

Methode E:

0,25 Mol des Diamins wurden gemeinsam mit 0,275 Mol Triethylamin in ca. 100 ml Aceton vorgelegt. Bei Raumtemperatur wurden 0,25 Mol des Säurechlorids aus Methode A in Form einer acetonischen Lösung zugetropft und das Reaktionsgemisch 4 Stunden am Rückfluß gekocht. Nach dem Abdestillieren des Lösungsmittels wurde der Rückstand mehrmals mit je 650 ml Wasser bei 50° ausgerührt, bis im abdekantierten Waschwasser keine Chlorionen mehr nachweisbar waren. Es wurde bei 60° im Vakuum getrocknet.

Methode F:

0,15 Mol des Diamins wurden gemeinsam mit 0,33 Mol Triethylamin in ca. 150 ml N-Methyl-pyrrolidon vorgelegt. Bei Raumtemperatur wurden 0,3 Mol des Säurechlorids aus Methode A zugetropft und der Tropftrichter mit 25 ml N-Methylpyrrolidon nachgespült. Es wurde 1 Stunde bei Raumtemperatur und 4 Stunden bei 50 - 60° nachgerührt, das Reaktionsgemisch in 1 l Wasser eingetragen und die gebildete harzige Fällung nach Abdekantieren der wäßrigen Phase noch mehrmals mit je 1 l Wasser von 50° ausgerührt, bis keine Chlorionen mehr nachgewiesen werden konnten. Trocknung bei 60°C im Vakuum.

Methode G:

0,15 Mol des Amins und 0,165 Mol Triethylamin wurden in 100 ml N-Methyl-pyrrolidon gelöst. 0,15 Mol des Säurechlorids aus Methode A wurden bei Raumtemperatur zugetropft und der Tropftrichter mit 25 ml N-Methyl-pyrrolidon nachgespült. Nach 4stündigem Erhitzen auf 50-60° wurde das Reaktionsgemisch auf 1 l Wasser gegeben und bei 50-60° verrührt. Von dem harzigen Produkt wurde abdekantiert und der Waschvorgang (bei 50-60°) mehrmals wiederholt, bis keine Chlorionen mehr nachweisbar waren. Wenn sich die Trennung der organischen von der wäßrigen Phase als schwierig und zeitraubend erwies, wurde der Ansatz durch Zentrifugieren aufgearbeitet. Vereinzelt bewirkte auch die Zugabe von etwas Säure eine leichtere Trennung der Phasen.
Trocknung bei 60° im Vakuum.

Methode H:

0,15 Mol der Amino-carbonsäure wurden in 75 ml 2n-Natronlauge gelöst und mit 50 ml Aceton versetzt. 0,15 Mol des Säurechlorids aus Methode A, verdünnt mit 50 ml Aceton wurden in 30 Minuten bei 20 bis 25° zugetropft und der Tropftrichter mit ca. 25 ml Aceton nachgespült. Gleichzeitig wurde aus einem zweiten Tropftrichter durch Zutropfen von ca. 75 ml 2n-Natronlauge der pH-Wert bei 8-8,5 (Glaselektrode) gehalten. Nach 3stündigem Nachrühren bei 20-25° wurde mit 400 ml Wasser verdünnt, mit konzentrierter Salzsäure kongosauer gestellt und das ausgefallene Reaktionsprodukt abgesaugt, $Cl^-$-Ionen frei gewaschen und bei 50-60° im Vakuum getrocknet.

Methode I:

0,22 Mol des Amins wurden in 50 ml Aceton gelöst und bei Raumtemperatur ($\leq$30°) mit 0,2 Mol des Säurechlorids nach Methode A, verdünnt mit 50 ml Aceton, in 15 Minuten tropfenweise versetzt. Nach 3stündigem Kochen am Rückfluß wurde das Lösungsmittel abdestilliert, der harzige Rückstand in ca. 1 l Wasser aufgenommen und mit ca. 17 g Natriumbicarbonat auf pH 7 gestellt. Die mit aliphatischen Aminen erhaltenen Reaktionsprodukte wurden mit Essigester ausgeschüttelt, mit Natriumsulfat getrocknet und im Rotationsverdampfer eingedampft. Die von aromatischen Aminen abgeleiteten Produkte konnten abgesaugt, frei von $Cl^-$-Ionen gewaschen und bei 60° im Vakuum getrocknet werden.

Methode J:

Eine Lösung von 0,1 Mol des Dialkylaminoamids in 200 ml Essigsäureethylester wurde mit 0,11 Mol Dimethylsulfat versetzt und bei Raumtemperatur 15 bis 20 Stunden verrührt. Das ausgefallene Reaktionsprodukt wurde abgesaugt, mit Essigester gewaschen und getrocknet.

Methode K:

0,15 Mol der Amino-sulfonsäure wurden mit der äquivalenten Menge 2n-Natronlauge in das Natriumsalz übergeführt, mit 50 ml Aceton verdünnt und mit 0,15 Mol des Säurechlorids nach Methode A, suspendiert in 50 ml Aceton, in 20 Minuten bei 20 bis 25° versetzt. Gleichzeitig wurde durch Zutropfen von 2n Natronlauge der pH-Wert bei 8-8,5 gehalten. Nach 4stündigem Verrühren bei Raumtemperatur wurde eingedampft

0103274

und der Rückstand im Vakuum bei 50-60° getrocknet. Die Diamino-stilben-disulfonsäure wurde analog unter Verwendung von 0,3 Mol Säurechlorid acyliert.

Anwendungsbeispiele:

Auf ein Gewebe aus Polyamid-6-Filamenten und Polyester wurden mit einem Foulard folgende Verbindungen mit einer Flottenaufnahme von 40-50 % aufgebracht. Die Produktmenge wurde so gewählt, daß ca. 0,125 % Auflage an Fluor auf den Geweben nach dem Trocknen sich befinden. Gearbeitet wurde aus acetonischen Lösungen, die ca. 1,2 g Substanz in 250 ml Aceton enthielt.

Die Gewebe wurden einmal luftgetrocknet und 1 Minute bei 160°C kondensiert. Die Ölabweisungswerte nach AATCC wurden direkt (AN), sowie nach 3 x Wäsche bei Kochtemperatur (3x Kw) in einer Waschmaschine geprüft.

Folgende Ölabweisungswerte wurden festgestellt:

| Lfd. Nr. nach Tabelle 1 | Polyamid | | | Polyester | | |
|---|---|---|---|---|---|---|
| | luftgetrocknet | 1 Min. 160°C AN | 3x Kw | luftgetrocknet | 1 Min. 160°C AN | 3x Kw |
| (101) | 6 | 6 | 4-5 | 6 | 6 | 6 |
| (111) | 6-7 | 6 | 6 | 6-7 | 6 | 5 |
| (114) | 6-7 | 6 | 6-7 | 6 | 6 | 5 |
| (115) | 6 | 5-6 | 5-6 | 6 | 5 | 6-7 |
| (116) | 6 | 6 | 6 | 6 | 6 | 4-5 |
| (117) | 7 | 5-6 | 6 | 7 | 6 | 4-5 |
| (118) | 6 | 6 | 6 | 6 | 6 | 5-6 |
| (119) | 6-7 | 5 | 6 | 7 | 6 | 6 |
| (121) | 6 | 5-6 | 4 | 6 | 5 | 5 |
| (124) | 6-7 | 6 | 4 | 6-7 | 6 | 7-8 |
| (131) | 6-7 | 6 | 6-7 | 6 | 6 | 7 |
| (135) | 6 | 6 | 5-6 | 6 | 6 | 5 |
| (136) | 6 | 6 | 5-6 | 6 | 6 | 4 |
| (140) | 6-7 | 6 | 7 | 6 | 6 | 6-7 |

Auf ein Wollgewebe wurden mit einem Foulard wäßrige Lösungen der unten angegebenen Verbindungen aufgebracht. Die Produktmenge wurde so gewählt, daß das Gewebe nach dem Trocknen jeweils 0,15 Gew.-% der Verbindungen, bezogen auf deren Fluor-Gehalt, enthielt. Die Gewebe wurden 4 Minuten bei 150°C kondensiert und dann die Werte für die Ölabweisung bestimmt nach der Testmethode Nr. 118-1966 der American Association of Textile Colorists and Chemists (AATCC). Die Ergebnisse sind in folgender Tabelle zusammengefaßt.

| Verbindung Nr. | Wert für die Ölabweisung |
|---|---|
| 301 | 5 |
| 302 | 4 - 5 |
| 311 | 2 - 3 |
| 305 | 5 |
| 306 | 5 |
| 310 | 4 |

Auf die gleiche Weise wurde ein Gewebe aus Polyacrylnitril mit der Verbindung Nr. 209 ausgerüstet. Der Wert für die Ölabweisung wurde dabei mit 5 gemessen.

Patentansprüche:

1. Perfluoralkyl-malein- und fumarsäureamide der Formel (1)

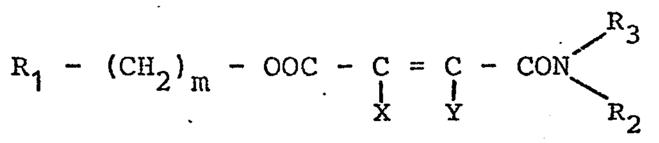

(1)

in welcher

$R_1$ Perfluoralkyl oder Perfluoralkoxy-perfluoralkyl mit jeweils insgesamt 2 - 20, vorzugsweise 4 - 14 C-Atomen,

$R_2$ Wasserstoff, geradkettiges oder verzweigtes $C_1$-$C_{18}$-Alkyl, das durch Hydroxylgruppen, eine Amino-, Alkylamino-, Dialkylamino- oder Trialkylammoniumgruppe, eine Acylaminogruppe, eine Aminoalkylaminogruppe, eine Acylaminoalkylaminogruppe, eine Alkoxy-, Hydroxyalkoxy-, Hydroxyalkylthiogruppe, eine Acyloxygruppe, 1-2 Carboxyl- oder funktionell abgewandelte Carboxylgruppen, eine Sulfo- oder funktionell abgewandelte Sulfogruppe, eine Phenyl-, Phenoxy- oder Furanylgruppe substituiert sein kann; $C_3$-$C_{18}$-Alkenyl, das durch Hydroxylgruppen substituiert sein kann; $C_3$-$C_8$-Alkinyl, das durch Hydroxylgruppen substituiert sein kann; Cyclohexyl, Phenyl, das durch 1 - 3 $C_1$-$C_8$ Alkylgruppen, 1 oder 2 Halogenatome, vorzugsweise Chloratome, eine Hydroxylgruppe, eine Amino-, Alkylamino-, Dialkylamino- oder Trialkylammoniumgruppe, eine Acylaminogruppe, eine Alkoxygruppe, eine Acyloxygruppe, 1-2 Carboxyl- oder funktionell abgewandelte Carboxylgruppen, 1-2 Sulfo- oder funktionell abgewandelte Sulfogruppen substituiert sein kann; α- oder ß-Naphthyl, das durch 1-3 Sulfogruppen substituiert sein kann; Phenylamino, das durch eine Sulfogruppe substituiert sein kann;

$R_3$ Wasserstoff, geradkettiges oder verzweigtes $C_1$-$C_8$-Alkyl, das durch Hydroxylgruppen, eine Sulfogruppe oder einen Phenylrest substituiert sein kann; Cyclohexyl,

$R_2$ und $R_3$ gemeinsam mit dem Stickstoffatom den Pyrrolidinring, Morpholinring, Piperidinring, Piperazinring oder Piperaziniumring,

X und Y Wasserstoff, Halogen oder $C_1$-$C_6$-Alkyl und

m eine ganze Zahl von 1 bis 6, vorzugsweise 1 bis 4

bedeuten.

2. Perfluoralkyl-malein- und -fumarsäureester der Formel (1) nach Anspruch 1 in welcher

$R_1$ eine Gruppe der Formel $C_lF_{2l+1}$-, eine Gruppe der Formel $H(C_2F_4)_n$- oder eine Gruppe der Formel $(CF_3)_2CFO(CF_2)_o$-

l die Zahlen 6, 8, 10, 12, 14,

n die Zahlen 1, 2, 3 und 4 und

o ganze Zahlen von 2 bis 8,

$R_2$ Wasserstoff, geradkettiges oder verzweigtes $C_1$-$C_8$-Alkyl, das durch Hydroxylgruppen, eine Amino-, Alkylamino-, Dialkylamino- oder Trialkylammoniumgruppe, eine Hydroxyalkoxygruppe, eine Carboxy- oder eine Sulfogruppe substituiert sein kann; Phenyl, das durch eine Hydroxylgruppe, eine niedere Alkoxygruppe eine niedere Dialkylamino- oder Trialkylammoniumgruppe, eine Carboxy- oder 1-2 Sulfogruppe substituiert sein kann; α- oder ß-Naphthyl-, das durch 1-3 Sulfo-Gruppen substituiert sein kann, eine Phenylaminogruppe,

$R_3$ Wasserstoff, geradkettiges oder verzweigtes $C_1$-$C_8$-Alkyl, das durch Hydroxylgruppen substituiert sein kann,

$R_2$ und $R_3$ gemeinsam mit dem Stickstoffatom den Morpholin-, Piperidino-, Piperazin- oder Piperaziniumring und

X und Y Wasserstoffatome, die vorzugsweise in Transstellung angeordnet sind,

bedeuten.

3. Verfahren zur Herstellung der Perfluoralkyl-malein- und -fumarsäureamide der Formel (1) nach Anspruch 1, dadurch gekennzeichnet, daß man 1 Mol Maleinsäureanhydrid mit einem Mol eines perfluorierten Alkohols oder Alkoxyalkohols der Formel

$$R_1 - (CH_2)_m - OH$$

umsetzt, den entstandenen Malein- und Fumarsäure-halbester in das Säurechlorid überführt und dieses, gegebenenfalls in Gegenwart eines tert. Amins, mit einem Amin der Formel

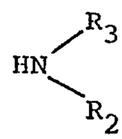

zur Reaktion bringt.

4. Verwendung der Perfluoralkyl-maleinsäure- und -fumarsäureamide nach Anspruch 1 zur öl- und wasserabweisenden Ausrüstung von Textilmaterialien.

Patentansprüche Österreich:

1. Verfahren zur Herstellung von Perfluoralkyl-malein- und -fumarsäureamiden der Formel (1)

$$R_1 - (CH_2)_m - OOC - \underset{X}{\underset{|}{C}} = \underset{Y}{\underset{|}{C}} - CON\begin{matrix} R_3 \\ R_2 \end{matrix} \quad (1)$$

in welcher

$R_1$ Perfluoralkyl oder Perfluoralkoxy-perfluoralkyl mit jeweils insgesamt 2 - 20, vorzugsweise 4 - 14 C-Atomen,

$R_2$ Wasserstoff, geradkettiges oder verzweigtes $C_1$-$C_{18}$-Alkyl, das durch Hydroxylgruppen, eine Amino-, Alkylamino-, Dialkylamino- oder Trialkylammoniumgruppe, eine Acylaminogruppe, eine Aminoalkylaminogruppe, eine Acylaminoalkylaminogruppe, eine Alkoxy-, Hydroxyalkoxy-, Hydroxyalkylthiogruppe, eine Acyloxygruppe, 1-2 Carboxyl- oder funktionell abgewandelte Carboxylgruppen, eine Sulfo- oder funktionell abgewandelte Sulfogruppe, eine Phenyl-, Phenoxy- oder Furanylgruppe substituiert sein kann; $C_3$-$C_{18}$-Alkenyl, das durch Hydroxylgruppen substituiert sein kann; $C_3$-$C_8$-Alkinyl, das durch Hydroxylgruppen substituiert sein kann; Cyclohexyl, Phenyl, das durch 1 - 3 $C_1$-$C_8$ Alkylgruppen, 1 oder 2 Halogenatome, vorzugsweise Chloratome, eine Hydroxylgruppe, eine Amino-, Alkylamino-, Dialkylamino- oder Trialkylammoniumgruppe, eine Acylaminogruppe, eine Alkoxygruppe, eine Acyloxygruppe, 1-2 Carboxyl- oder funktionell abgewandelte Carboxylgruppen, 1-2 Sulfo- oder funktionell abgewandelte Sulfogruppen substituiert sein kann; α- oder ß-Naphthyl, das durch 1-3 Sulfogruppen substituiert sein kann; Phenylamino, das durch eine Sulfogruppe substituiert sein kann;

$R_3$ Wasserstoff, geradkettiges oder verzweigtes $C_1$-$C_8$-Alkyl, das durch Hydroxylgruppen, eine Sulfogruppe oder einen Phenylrest substituiert sein kann; Cyclohexyl,

$R_2$ und $R_3$ gemeinsam mit dem Stickstoffatom den Pyrrolidinring, Morpholinring, Piperidinring, Piperazinring oder Piperaziniumring,

X und Y Wasserstoff, Halogen oder $C_1$-$C_6$-Alkyl und

m eine ganze Zahl von 1 bis 6, vorzugsweise 1 bis 4 bedeuten,

dadurch gekennzeichnet, daß man 1 Mol Maleinsäureanhydrid mit einem Mol eines perfluorierten Alkohols oder Alkoxyalkohols der Formel

$$R_1 - (CH_2)_m - OH$$

umsetzt, den entstandenen Malein- und Fumarsäure-halbester in das Säurechlorid überführt und dieses, gegebenenfalls in Gegenwart eines tert. Amins, mit einem Amin der Formel

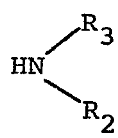

zur Reaktion bringt.

2. Verfahren nach Anspruch 1, dadurch gekennzeichnet, daß man Perfluoralkyl-malein- und -fumarsäureamide der Formel (1) herstellt, worin

$R_1$ eine Gruppe der Formel $C_lF_{2l+1}$-, eine Gruppe der Formel $H(C_2F_4)_n$- oder eine Gruppe der Formel $(CF_3)_2CFO(CF_2)_o$-

l die Zahlen 6, 8, 10, 12, 14,

n die Zahlen 1, 2, 3 und 4 und

o ganze Zahlen von 2 bis 8,

$R_2$ Wasserstoff, geradkettiges oder verzweigtes $C_1$-$C_8$-Alkyl, das durch Hydroxylgruppen, eine Amino-, Alkylamino-, Dialkylamino- oder Trialkylammoniumgruppe, eine Hydroxyalkoxygruppe, eine Carboxy- oder eine Sulfogruppe substituiert sein kann; Phenyl, das durch eine Hydroxylgruppe, eine niedere Alkoxygruppe eine niedere Dialkylamino- oder Trialkylammonium-

gruppe, eine Carboxy- oder 1-2 Sulfogruppe substituiert sein kann; α- oder ß-Naphthyl-, das durch 1-3 Sulfogruppen substituiert sein kann, eine Phenylaminogruppe,

$R_3$ Wasserstoff, geradkettiges oder verzweigtes $C_1$-$C_8$-Alkyl, das durch Hydroxylgruppen substituiert sein kann,

$R_2$ und $R_3$ gemeinsam mit dem Stickstoffatom den Morpholin-, Piperidino-, Piperazin- oder Piperaziniumring und

X und Y Wasserstoffatome, die vorzugsweise in Transstellung angeordnet sind,

bedeuten.

3. Verwendung der Perfluoralkyl-maleinsäure- und -fumarsäureamide nach Anspruch 1 zur öl- und wasserabweisenden Ausrüstung von Textilmaterialien.